# EUROPEAN PATENT APPLICATION

(11) **EP 1 721 706 A1**
(43) Date of publication of application: **15.11.2006**
(21) Application number: 06425315.6
(22) Date of filing: 10.05.2006
(51) Int. Cl.: B25B 7/02

(54) **Tool for graduated modelling of biocompatible plates used in orthognadontic surgery**

(30) Priority: 11.05.2005 IT MC20050043
(71) Applicant: PIERGIACOMI SUD - S.R.L., 63033 Monteprandone (AP) (IT)
(72) Inventor: Di Emidio, Paolo, 64010 Controguerra (TE) (IT)
(74) Representative: Baldi, Claudio

(57) **Abstract**

The present invention refers to a tool used for graduated modelling of biocompatible plates used in orthognadontic surgery, of the type composed of a pair of claws mutually hinged and subjected to the action of an intermediate spring, characterised in that the end of the claws (1a) are provided with tips with stepped or "S" profiles in asymmetrical opposite position, capable to provide perfect prismatic matching when they touch.

## Description

The present patent application for industrial invention refers to a tool used for graduated modelling of biocompatible plates used in orthognadontic surgery and, more precisely, in maxillary osteosynthesis and advancement genioplasty.

In the last few years therapy of face skeleton anomalies has become one of the most active and interesting sectors of maxillo-facial surgery, due to the development of new surgery techniques that allow to move any portion of the craniofacial skeleton in any plane of space without visible incision.

A surgical operation of this kind is executed to correct a facial dental-skeletal dismorphy, that is to say a condition in which the maxillary bone bases are connected in a more or less altered way; in particular, such an anomaly may affect the maxillary base only, the jaw only or both the stone bases.

Investigations have shown that similar maxillary alterations are caused by different factors: mainly bad habits (such as thumb sucking in childhood), alterations of tongue shape and position and genetic factors.

Due to the variety of clinical, occlusal and skeletal characteristics of the different cases, a careful diagnostic study is necessary in order to plan the ideal surgical treatment.

The diagnostic study must be performed according to the following modes:
- aesthetical, clinical and photometric analysis of the patient's facial bones, both front and side;
- occlusal examination
- realization of plaster arch moulds, which are mounted on suitable articulators
- cranial teleradiography in lateral and antero-posterior projection and orthopantogram
- cephalometric investigation.

In particular, the cephalometric investigation plays a very important role, since it is used to evaluate the spatial ratios between maxillary and jaw, both mutually and with relation to the base of the skull.

Based on the latter clinical examination, and without neglecting the findings of the other investigations, a simulation is carried out to determine the bone segment to be moved (maxillary and/or jaw), and in particular the number of millimetres.

For instance, if the maxillary is retracted by 3 millimetres, it will be moved forward by the same number of millimetres; the same is also true for the jaw.

The first phase of the treatment is orthodontic (more exactly, pre-surgical orthodontics) and aims at re-aligning the arches, through the elimination of dental compensations and restoration of normal dental-basal ratios; orthodontic treatment on two levels is sometimes necessary for the upper arch.

In case of anomalies by defect in antero-posterior direction of the middle third of the facial skeleton, surgical therapy consists in the correct repositioning of the upper maxillary, following to the execution of specific osteotomy.

Evidently, the decision shall be determined by the plane of treatment. For example, osteotomy known as "Le Fort I osteotomy" is used for upper maxillary, when it is necessary to correct the posterior vertical excess.

A segmentary osteotomy or transversal expansion is executed in case of contraction of the upper arch.

With reference to the jaw, the most common operation is the saggiatal osteotomy of the mandibular branches, according to the technique known as "Obwegeser, Dal Pont or Gotte". This technique is appreciated for its versatility, since it allows for corrections in postero-anterior direction and in other spatial planes, and for the large contact areas between bone segments.

During the execution of the said surgical operations, the upper maxillary is fixed by means of titanium plates that need to be modelled by the surgeon by hand.

To this end, manufacturing companies provide kits of titanium plates that are perfectly flat and can be given different shapes ("L", "Y"- shaped, etc.). Because of this, the surgeon needs to model (or better said, "curve") the plates in the operating room according to the specific requirements of each patient, and then fix them with suitable screws to obtain osteotomy.

However, the shape given to the plate by the surgeon is not perfect and therefore is not perfectly suitable for the specific requirements.

If, for example, the upper maxillary must be moved by 3 mm, the surgeon should bend the plate by 3 mm exactly, and this is extremely difficult to do empirically.

No specific tools are so far available, and surgeons are forced to use improper tools (such as Klemmer forceps) that are typically designed for different uses.

The purpose of the present invention is to provide for the first time a tool expressly designed to be used by surgeons to model titanium plates practically and precisely.

The general structure of the new tool of the invention basically reproduces a traditional surgical forceps, with an innovative peculiarity consisting in the presence of two identical, perfectly cooperating tips on the claws.

More precisely, each tip has a stepped or "S" cross-section, since it consists in two adjacent sections with different height connected by means of an inclined intermediate profile.

The two tips can match perfectly, since the section with higher height of one tip is matched with the section with lower height of the other tip and since the inclined intermediate profiles of the two tips have the same inclination.

The two tips operate as the jaws of traditional clamps, with the only difference that their cooperating areas are shaped as an "S", and not flat, in order to provide prismatic, not front, matching.

Thanks to the special configuration of the tips of the tool according to the present invention, the titanium plate is inserted and tightened between the tips of the tool, thus losing its original perfect planarity and assuming a stepped or "S" shaped, according to the profile given to the cooperating areas of the two tips.

For purposes of clarity the description of the invention continues with reference to the enclosed drawing, which is intended for purposes of illustration only and not in a limiting sense, whereby:
- figures 1 and 2 are a front and a side view of the forceps of the invention, respectively;
- figure 3 is a diagrammatic view that consists in a top view of the tips of the forceps.

With reference to the aforementioned figures, the new tool of the invention (1) has basically the same general structure of a surgical forceps, with two claws (1a) mutually hinged and subjected to the action of an intermediate spring (1b).

The end of the claws (1a) are provided with metal tips (2, 3) in perpendicular position, with stepped internal profiles in asymmetrical opposite position.

In particular, the first tip (2) has a section (2a) with higher thickness and higher width, which is connected with an adjacent section (2c) with lower thickness and lower width by means of an inclined intermediate section (2b).

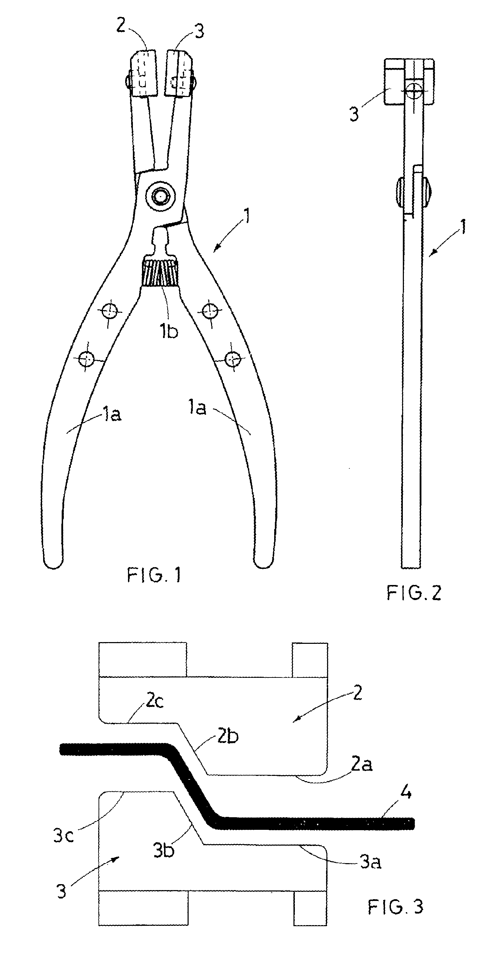

As shown in figure 3, the second tip (3), designed to be opposed to the first tip (2), has a asymmetrical profile that provides perfect prismatic matching of the two tips (2, 3) when the claws (1a) of the tool are tightened.

As shown in figure 3, the second tip (3) has a (3a) with lower thickness and higher width, which is connected with an adjacent section (3c) with higher thickness and lower width by means of an inclined intermediate section (3b).

The width of the first section (2a) of the first tip (2) and the width of the first section (3a) of the second tip (3) are perfectly identical; the same is also true for the width of the third section (2c) of the first tip (2) and the third section (3c) of the second tip (3).

Likewise, the thickness of the first section (2a) of the first tip (2) and the thickness of the third section (3c) of the second tip (3) are perfectly identical; the same is also true for the thickness of the third section (2c) of the first tip (2) and the thickness of the first section (3c) of the second tip (3).

The titanium plate (4) is tightened between the two tips (2, 3) and is exactly modelled according to the "stepped" contact line between the tips (2, 3).

In practical terms, each titanium plate is given the same stepped or "S" profile, with two rectilinear sections (4a, 4c) at two different heights and connected by means of an inclined intermediate section (4b); it being provided that the difference in height between the two rectilinear sections (4a, 4c) and consequently the bending angle of the plate (4) perfectly correspond to the difference in height between the "flat" sections (2a/2c, 3a/3c) of the two tips (2, 3).

Therefore, the tool of the invention allows for perfect modelling of titanium plates, eliminating bending inaccuracies when the plates are manually bent by the surgeon.

According to the same inventive idea, the tool of the invention may be realised in different alternative embodiments, each of them provided with tips capable of giving the plates (4) specific bending angles in the same stepped or "S" profile.

More precisely, although they maintain the same structure, the different tips (2, 3)have a different height between the flat sections (2a/2c, 3a/3c).

In order to provide a solution to the surgeon's different requirements, an alternative embodiment of the invention may be provided with interchangeable tips, meaning that each pair of tips can be removed and replaced with other pairs of tips provided with different dimensional characteristics.

## Claims

1. Tool for graduated modelling of biocompatible plates used in orthognadontic surgery, of the type composed of a pair of claws (1a) mutually hinged and subjected to the action of an intermediate spring (1b), **characterised in that** the end of the claws (1 a) are provided with tips (2, 3) with stepped or "S" profiles in asymmetrical opposite position, capable to provide perfect prismatic matching when they touch.

2. Tool as defined in claim 1, **characterised in that** the stepped profile given to the tips (2, 3) is such that the first tip (2) has a section (2a) with higher thickness and higher width, which is connected with an adjacent section (2c) with lower thickness and lower width by means of an inclined intermediate section (2b), while the second tip (3), designed to be opposed to the first tip (2), has a section (3a) with lower thickness and higher width, which is connected with an adjacent section (3c) with higher thickness and lower width by means of an inclined intermediate section (3b); it being provided, in particular, that the width of the first section (2a) of the first tip (2) and the width of the first section (3a) of the second tip (3) are perfectly identical and the width of the third section (2c) of the first tip (2) is identical to the width of the third section (3c) of the second tip (3) and, likewise, the thickness of the first section (2a) of the first tip (2) and the thickness of the third section (3c) of the second tip (3) are identical and the thickness of the third section (2c) of the first tip (2) and the thickness of the first section (3c) of the second tip (3) are identical.

3. Tool as defined in the first or both claims, **characterised in that** the tips (2, 3) are connected to the claws (1a) with connection means that can be removed quickly and easily.
